# EUROPEAN PATENT SPECIFICATION

(11) **EP 3 030 283 B1**
(45) Date of publication and mention of the grant of the patent: **18.03.2020**
(21) Application number: 14750904.6
(22) Date of filing: 29.07.2014
(51) Int. Cl.: A61M 1/16, A61M 1/28, A61J 1/10, A61J 1/16, F24V 30/00

(54) **SELF-WARMING PERITONEAL DIALYSIS SOLUTION BAG**
SELBSTERWÄRMENDER BEUTEL FÜR PERITONEALDIALYSELÖSUNG
POCHE DE SOLUTION DE DIALYSE PÉRITONÉALE AUTO-CHAUFFANTE

(30) Priority: 06.08.2013 US 201313959797
(43) Date of publication of application: 15.06.2016
(73) Proprietor: Fresenius Medical Care Holdings, Inc., Waltham, MA 02451 (US)
(72) Inventor: BARONE, Joseph D., Jr., Warrendale, PA 15086 (US); CONLON, Andrea M., Warrendale, PA 15086 (US); BARRON, John Albert, III, Warrendale, PA 15086 (US); NIESSLEIN, Michael David, Warrendale, PA 15086 (US)
(74) Representative: Grünecker Patent- und Rechtsanwälte PartG mbB
(86) International application number: PCT/US2014/048521
(87) International publication number: WO 2015/020828

(56) References cited:
- EP-A1- 0 950 422
- EP-A2- 1 164 092
- DE-A1- 2 454 482
- DE-U1- 9 308 204
- US-A1- 2004 118 477
- US-A1- 2004 247 016
- US-A1- 2009 009 179
- US-A1- 2010 146 849
- US-A1- 2011 000 902
- US-B1- 6 289 889

## Description

### FIELD OF THE INVENTION

The purpose of this concept is to be able to heat dialysis solution within the bag itself and eliminating the need for an external power/heating source. This is to be done with an air-activated layer attached to the bag that will heat it through an exothermic chemical reaction (via iron oxidation).

### BACKGROUND OF THE INVENTION

Currently, patients and/or clinicians are using an electric heating pad and cooler in order to heat the dialysis solution to the necessary 37°C (98.6°F) before putting into the patient's peritoneal cavity. The bag currently holds 1500 milliliters of solution and has rough dimensions of 20.32 cm (8.00 inches) wide by 27.94 cm (11.00 inches) long, with thickness of 3.81 cm (1.50 inches). Prior art systems for patients requiring hemodialysis or peritoneal dialysis involve pumping a large volume of dialysate through a dialyzing device. In these prior art devices, the used dialysate is then discarded.
As of now, patients must have an available power source nearby, as well as a heating pad and cooler if possible. This limits the patient to their location where they perform the treatment as well as having these two items to do the heating. However, with the new Wearable Artificial Kidney (WAK) being developed by FMC/Renal Solutions, it is a peritoneal dialysis system with the power, filtration, and pumping all contained within a vest. With this system, they patient has the freedom to go almost anywhere but would be limited by the heating of the solution. U.S. Patent No. US 6,289,889 B1 describes a heater or self-heating package that generates heat by contacting a heat-producing composition with an activating solution. The heater is activated by rupturing a frangible seal to allow the heater components to mix.

### SUMMARY OF THE INVENTION

The present invention provides a self-warming peritoneal dialysis solution bag as defined in claim 1.

According to one or more embodiments of the present disclosure, a self-warming peritoneal dialysis solution bag is provided that contains a peritoneal dialysis solution. The bag has an outer surface and a non-electric, warming patch affixed to the outer surface. The non-electric, warming patch can include an activatable agent that undergoes an exothermic reaction when activated. The warming patch can retain a sufficient amount of activatable agent to generate enough heat, upon activation, to warm the contents of the bag to at least 35°C (95°F).

According to one or more embodiments of the present disclosure, a system is provided and comprises a peritoneal dialysis solution bag and a non-electric, warming patch configured to be adhered to the bag. The non-electric, warming patch can include an activatable agent that undergoes an exothermic reaction when activated. The warming patch can retain a sufficient amount of activatable agent to generate enough heat, upon activation, to warm the contents of the bag to at least 35°C (95°F).

According to one or more embodiments of the present disclosure, a kit is provided for warming a peritoneal dialysis solution bag. The kit can include instructions for use, and a non-electric, warming patch configured to be affixed to an outer surface of a peritoneal dialysis solution bag. The warming patch can include an activatable agent that undergoes an exothermic reaction when activated. The warming patch can retain a sufficient amount of activatable agent to generate enough heat, upon activation, to warm the contents of the peritoneal dialysis solution bag to at least 35°C (95°F). A packaging container can be included as part of the kit and can be configured to contain the non-electric, warming patch. The kit can also include a thermometer configured to be adhered or affixed to the solution bag for monitoring the temperature of the contents of the solution bag. The kit can also include a reflector configured to be adhered or affixed to the solution bag for reflecting heat into the solution bag.

According to one or more embodiments of the present disclosure, a method is provided for warming a peritoneal dialysis solution bag. The method can comprise contacting a peritoneal dialysis solution bag containing peritoneal dialysis solution, with a non-electric, warming patch. The warming patch can contain an activatable agent that undergoes an exothermic reaction upon activation. The method can include activating the activatable agent to cause the exothermic reaction, forming an activated patch. The method can further include heating the peritoneal dialysis solution in the peritoneal dialysis solution bag, with the activated patch. The method can also include monitoring the temperature of the contents of the bag using a thermometer adhered or affixed to a surface of the solution bag.

### BRIEF DESCRIPTION OF THE DRAWINGS

The invention may be more fully understood with reference to the accompanying drawings. The drawings are intended to illustrate, not limit, the present teachings.
FIG. 1 is a perspective view of a self-warming peritoneal dialysis solution bag according to one or more embodiments of the present disclosure.
FIG. 2 is a top perspective view of a warming patch according to one or more embodiments of the present invention, which can be used on a peritoneal dialysis solution bag to warm solution contained in the bag.
FIG. 3 is a cross-sectional view of the warming patch shown in FIG. 2, taken along line III-III shown in FIG. 2.
FIG. 4 is a top view of a peritoneal dialysis solution bag including a two-compartment warming patch, according to one or more embodiments of the present disclosure.
FIG. 5 is a side view of the peritoneal dialysis solution bag and warming patch shown in FIG. 4, which also shows a heat-reflective metal foil adhered to a bottom surface of the bag.
FIG. 6 is a perspective schematic view of a self-warming peritoneal dialysis solution bag according to the present disclosure, showing exemplary dimensions, in inches, of a solution bag and a warming patch.
FIG. 7 is a perspective view of a kit according to one or more embodiments of the present disclosure, showing the packaging box and the instructions, warming patch, reflective foil, and thermometer that are packaged in the box.

### DETAILED DESCRIPTION OF THE INVENTION

According to one or more embodiments of the present disclosure, a self-warming peritoneal dialysis solution bag is provided. The bag contains a peritoneal dialysis solution and has a non-electric, warming patch affixed to an outer surface thereof. The non-electric, warming patch can retain, contain, or comprise an activatable agent that undergoes an exothermic reaction when activated. The warming patch can retain a sufficient amount of activatable agent to generate enough heat, upon activation, to warm the contents of the bag to at least 35°C (95°F). The contents of the bag can include a sufficient amount of peritoneal dialysis solution to perform a peritoneal dialysis therapy on a user. The warming patch can comprise a removable protective cover that prevents activation of the activatable agent until the removable protective cover is removed. The removable protective cover can comprise an adhesive release liner, a sealed layer, a frangible member, or the like. The removable protective cover can be adhered or otherwise affixed to the non-electric, warming patch. The removable protective cover can be removably adhered to the non-electric, warming patch by a releasable pressure sensitive adhesive. In some cases, the activatable agent can be activated by contact with air, water, or both, to cause the exothermic reaction. In some cases, the activatable agent can be activated by contact with a second agent, a reactant, a reagent, a catalyst, a promoter, an initiator, a combination thereof, or the like. The activatable agent can comprise an oxidizable metal, for example, an oxidizable metal comprising iron, an iron oxide material, or a combination thereof.

According to one or more embodiments of the present disclosure, the non-electric, warming patch can comprise two or more compartments, each being separated from one or more other compartments by a frangible seal. The compartments can contain respective reactants, and when the reactants are brought together, for example, by rupturing the frangible seal, an exothermic reaction results that can be used to heat the contents of the peritoneal dialysis solution bag. A first compartment of the two or more compartments can comprises a first reactant, a second compartment of the two or more compartments can comprise a second reactant, and when the first and second reactants come into contact with each other, they exothermically react. At least one of the compartments can contain a sodium acetate reagent.

One or more surfaces or surface areas of the self-warming peritoneal dialysis solution bag, for example, an outer surface, can be in contact with a heat-reflective material. The heat-reflective material can comprise a metal or other heat-reflective foil adhered to an outer surface of the bag. The heat-reflective foil can be in contact with a first side of the bag, the heat-reflective foil can comprise a reflective surface that faces the inside of the bag, and the non-electric, warming patch can be disposed on a second side of the bag, opposite the first side.

Two or more warming patches can be provided on a single bag. If two or more warming patches are provided, each can comprise its own removable protective cover. If two warming patches are included, then two removable protective covers can respectively be provided. Each of the removable protective covers can protect a respective one of the two or more warming patches. One of the two or more removable protective covers can be removed to activate one warming patch without removing one or more of the other protective covers and thus without activating one or more of the other warming patches.

A temperature indicator or thermometer can be affixed or adhered to the peritoneal dialysis solution bag, for example, adhered to an outer surface of the bag. The temperature indicator can comprise a color indicator, a liquid crystal display, a combination thereof, or the like. If a color indicator is provided, the color indicator can comprise a first color indicator configured to indicate when the solution in the bag is at a suitable temperature. One or more other color indicators can be provided and configured to indicate when the solution in the bag is not hot enough, too hot, or both. The temperature indicator can comprise a flexible liquid crystal display adhered to an outer surface of the bag in the same manner as similar thermometers are stuck to an outside surface of an aquarium.

According to one or more embodiments of the present disclosure, a combination of a self-warming peritoneal dialysis solution bag and a bag holder is provided. The bag holder can be configured to be worn by a user and can comprise a pouch configured to accommodate the self-warming peritoneal dialysis solution bag. The pouch can comprise heat-insulating material configured to thermally insulate the peritoneal dialysis solution bag. The bag holder can comprise a vest, for example, that has through-holes for accommodating a user's arms. The bag holder can comprise a belt, waist band, a hip-pack, or a combination thereof.

According to one or more embodiments of the present disclosure, a system is provided that comprises a peritoneal dialysis solution bag and a non-electric, warming patch configured to be adhered to the bag. The non-electric, warming patch can be provided separately from the bag and can be adhered, affixed, or otherwise made to be in contact with the bag, when desired. The non-electric, warming patch can be as described herein, and can comprise, for example, an agent that undergoes an exothermic reaction when activated. The warming patch can retain a sufficient amount of activatable agent to generate enough heat, upon activation, to warm the contents of the bag to at least 35°C (95°F). The non-electric, warming patch can comprise an adhesive surface configured to be adhered to the bag, and a removable protective liner on the adhesive surface. The removable protective liner can be left on the adhesive surface until the warming patch is ready to be used, at which time the removable protective liner can be removed from the adhesive surface so the warming patch can be adhered to the bag. The non-electric, warming patch can comprise two compartments that are separated from one another by a frangible seal, as described herein.

According to various embodiments of the present disclosure, a kit is provided that can comprise instructions for warming a peritoneal dialysis solution bag, and a non-electric warming patch configured to be adhered, affixed, or otherwise made to be in contact with an outer surface of a peritoneal dialysis solution bag. The kit can be provided separate from a peritoneal dialysis solution bag and can be tailored to warm a specific bag of a specific volume in a specific climate. The warming patch can comprise an activatable agent that undergoes an exothermic reaction when activated, as described herein. The warming patch can comprise a sufficient amount of activatable agent to generate enough heat, upon activation, to warm the contents of a specified peritoneal dialysis solution bag to at least 35°C (95°F). The contents of the kit can be packed together in a packaging container such as a box, bag, pouch, or other packaging. The instructions can be provided on an instruction sheet and the instruction sheet can be contained in the packaging container. The instructions can be printed on the packaging container. The packaging container can comprise a sealed box and the sealed box can be wrapped with a wrapper. The packaging container can be hermetically sealed. Different kits can be provided for warming different solution bags.

The kit can further comprise a thermometer, a heat-reflective foil, or both. If provided, the thermometer and heat-reflective foil can be contained in the packaging container. The thermometer can comprise color indicators including a color indicator configured to indicate that the temperature of contents of the peritoneal dialysis solution bag are within an acceptable temperature range for taking a more accurate temperature, or for transfer into a peritoneal cavity. The non-electric, warming patch can comprise an adhesive surface configured to be adhered to a peritoneal dialysis solution bag, and a removable protective liner disposed on, and protecting, the adhesive surface.

In yet other embodiments of the present disclosure, a method is provided that comprises contacting a peritoneal dialysis solution bag containing peritoneal dialysis solution, with a non-electric, warming patch. The warming patch can contain an activatable agent that undergoes an exothermic reaction upon activation, as described herein. The method can further comprise activating the activatable agent to cause the exothermic reaction and thus form an activated patch. The method can include heating the peritoneal dialysis solution in the peritoneal dialysis solution bag, with the activated patch. The heating can be sufficient to heat the solution in the bag to at least 35°C (95°F). The method can also include monitoring a color-indicator thermometer attached to the peritoneal dialysis solution bag to determine whether the temperature of the solution in the bag is within an appropriate range for use. The method can include adhering the warming patch to the peritoneal dialysis solution bag prior to activating the activatable agent, or after activating the activatable agent. As described herein, the warming patch can comprise two compartments separated by a frangible seal, and the activating can comprise rupturing the frangible seal and mixing together components from both compartments.

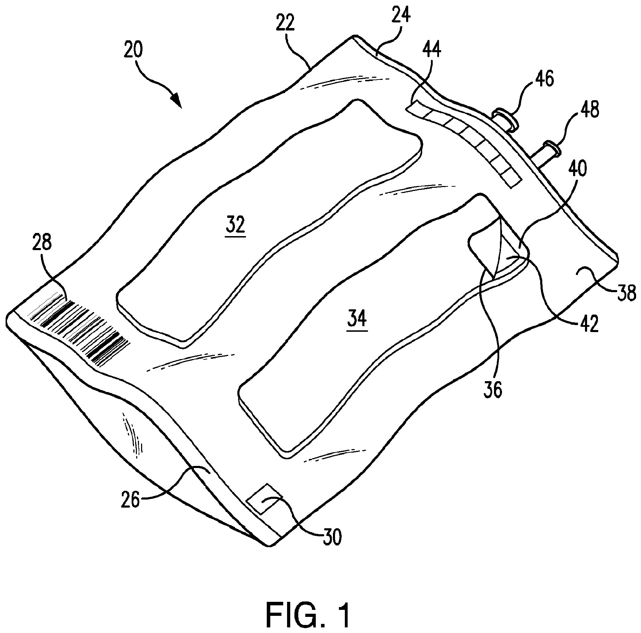

With reference now to the drawings, FIG. 1 shows and exemplary self-warming peritoneal dialysis solution bag 20 according to the present disclosure. The material for bag 20 can include a sidewall 22 comprising a sterile plastic material, for example, a polyvinyl chloride (PVC) material, a polyolefinic material, a polyalkylene material, or the like. Bag 20 can be heat-sealed at its ends 24 and 26. Bag 20 can include a barcode 28, a radio frequency identification tag 30, and/or other identifying indicia, markings, and information. Labels, information, instructions, codes, and tags on the bag can be machine-readable. Bag 20 can be provided with one or more warming members, and in the embodiment shown in FIG. 1, two warming members are provided, warming members 32 and 34. Each warming member 32 and 34 can include a warming material that can be activated by exposure to air, oxygen, water, humidity, a combination thereof, and/or one or more other environmental element. Each warming member 32 and 34 can include a removable protective cover 36 that can be adhered to a first surface 38 of bag 20, for example, by a bead of adhesive 40. When peeled away, protective cover 36 exposes a mesh, porous, permeable, semi-permeable, or other breathable membrane 42 that is configured to retain an activatable warming material. The warming material can comprise, for example, a powder material, a metal powder, a fibrous material, an oxidizable metal, and/or a chemical agent that can exothermically react. Other components can also be provided in the warming material, for example, fillers, insulative particles, heat-distributing particles, heat retaining particles, and combinations thereof. Breathable material 42 can be configured to have openings that are not larger than the smallest particles to be retained. As can be seen, protective cover 36 can be removed from warming member 34 while the protective cover of warming member 32 can remain intact.

Peritoneal dialysis solution bag 20 can also include a thermometer 44 configured to display the temperature of the solution contained by bag 20, and/or to otherwise indicate whether the solution is within an appropriate temperature range to be drained into a peritoneum. Thermometer 44 can also indicate whether the temperature of the solution in bag 20 is too hot, too cold, or both. In an exemplary embodiment, thermometer 44 can comprise graduations that include one or more graduations at a first end of the thermometer, which turn blue if the temperature of the solution is too cold. Thermometer 44 can include one or more graduations at a second end thereof, which turn red if the temperature of the solution is too hot. Moreover, thermometer 44 can include one or more graduations in a middle portion thereof, which turn green when the temperature of the solution is within an appropriate temperature range for use.

In use, a user can activate one or more warming members or warming patches by removing the respective protective cover. The user can then monitor the thermometer until it indicates that the temperature of the solution is within an appropriate temperature range. Once the temperature of the solution reaches an appropriate range, the user can then either remove the activated warming member or seal the warming member so that the warming material inside is no longer in contact with the environment and the exothermic reaction ceases. Protective cover 36 can, for example, be reapplied to adhesive bead 40 to seal off warming member 34 once an adequate temperature of the solution is reached.

Peritoneal dialysis solution bag 20 can also be provided with a drainage port 46 through which solution can pass into a delivery tube (not shown). A medication port 48 can also be provided so that a medication can be added to the solution within bag 20 before the solution is drained into a peritoneum.

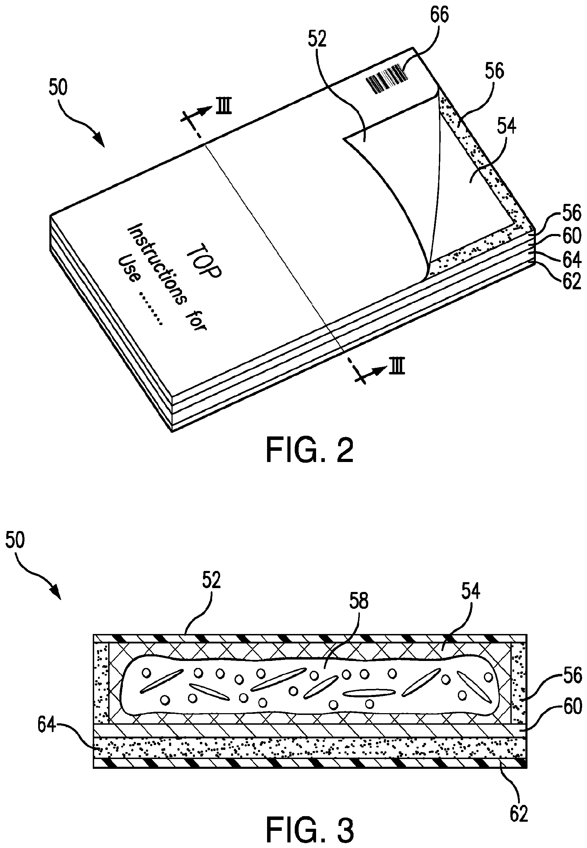

FIG. 2 is a top perspective view of a warming patch 50 that can be used on a peritoneal dialysis solution bag, in accordance with one or more embodiments of the present invention. Patch 50 can comprise a multi-layered structure including a removable protective cover 52 that can be peeled away to expose a breathable membrane 54 containing a warming material 58 as shown in FIG. 3. Removable protective cover 52 can protect breathable membrane 54 and warming material 58 from exposure to environmental elements. Removable protective cover 52 can be adhered to a base layer 60 by an adhesive bead 56 provided at least around the periphery of warming patch 50. Base layer 60 can comprise, for example, a polymeric film or layer. Base layer 60 can comprise a PVC material, a polyolefin material, a polyalkylene material, or the like.

FIG. 3 is a cross-sectional view taken along line III-III shown in FIG. 2. As can best be seen in FIG. 3, breathable membrane 54, and warming material 58 retained therein, can be sealed and thus protected from the environment by polymeric layer 60, adhesive bead 56, and top removable protective cover 52. All layers and materials of the warming patch should be capable of withstanding the heat generated by the exothermic reaction of warming material 58, without melting or degrading.

Warming patch 50 can be adhered to a peritoneal dialysis solution bag (not shown) by an adhesive layer 64 applied to a bottom surface of base layer 60. Adhesive layer 64 can be protected until it is needed for use, by a bottom, removable, protective liner 62. Warming patch 50 can be applied to a peritoneal dialysis solution bag by removing protective liner 62 and adhering the resulting exposed adhesive layer 64 to a surface of the solution bag. Once applied to a solution bag, warming patch 50 can otherwise remain intact such that warming material 58 remains protected from environmental elements. Anytime after applying warming patch 50 to a solution bag, removable protective cover 52 can be removed from the patch to expose breathable membrane 54, and thus warming material 58, to environmental elements, thus activating an exothermic reaction of warming material 58. The environmental elements can include air, oxygen, moisture, water, or the like, which activate warming material 58. As an example, if warming material 58 includes an oxidizable metal powder such as iron, removing protective cover 52 can cause warming material 58 to be exposed to oxygen in the environment, thus activating an exothermic reaction whereby iron is oxidized to form iron oxide.

Although FIG. 2 depicts adhesive bead 56 remaining attached to base layer 60 when removable protective cover 52 is peeled away, it is also within the scope of the present teachings to provide an adhesive bead that remains attached to removable protective cover 52 and thus is peeled away from base layer 60 along with removable protective cover 52. Although not shown, breathable membrane 54 can be adhered or otherwise fixed or secured to one or more base layers of warming patch 50.

As shown in FIG. 2, warming patch 50 can be provided with a bar code 66, an expriation date, a lot number, a listing of contents, other labeling, other indicia, one or more other tags, other information, combinations thereof, and the like.

Warming patch 50 can be packaged and sold separately, or together with, a peritoneal dialysis solution bag. To monitor the temperature of the solution within such a solution bag, a thermometer can also be provided, for example, a flexible liquid crystal temperature indicator such as any of models C-8701, C-8702, or C-8704 available from American Thermal Instruments (ATI) of Dayton, Ohio. The thermometer can be reusable, single-use, disposable, a combination thereof, or the like. One or more thermometers can be packaged together with one or more warming patches so that a thermometer and warming patch can both be applied to a solution bag for warming and monitoring the temperature thereof. The present invention also provides a kit wherein at least one warming patch and at least one thermometer are packaged together, for example, in a sealed box, bag, package, or other container.

The layer thicknesses depicted in FIGS. 2 and 3 are not drawn to size and have been exaggerated for the purpose of illustrating the present invention.

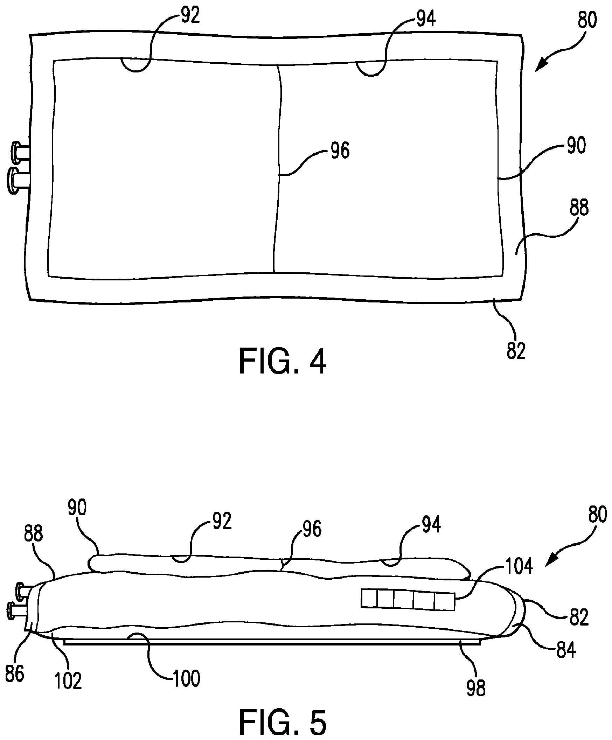

FIGS. 4 and 5 depict another exemplary embodiment of the present disclosure, wherein the warming patch comprises two compartments separated by a frangible seal that can be ruptured so that the contents of the two compartments can mix and cause an exothermic reaction. A self-warming peritoneal dialysis solution bag 80 comprises a peritoneal dialysis solution bag 82 and a two-compartment warming patch 90 adhered to a top surface 88 thereof. A heat-reflective foil 98 is adhered to a bottom surface 102 of peritoneal dialysis solution bag 82. Although FIGS. 4 and 5 show two-compartment warming patch 90 and heat-reflective foil 98 already adhered to peritoneal dialysis solution bag 82, it is to be understood that warming patch 90, heat-reflective foil 98, and thermometer 104 can be packaged and/or provided separately from a peritoneal dialysis solution bag and subsequently applied to the bag.

As best seen in FIG. 5, peritoneal dialysis solution bag 82 is heat sealed at a first end 84 thereof, and heat sealed at a second end 86 thereof. Two-compartment warming patch 90 can be affixed or adhered to a smooth surface of peritoneal dialysis solution bag 82 such that it is in continuous and intimate contact with the bag. A layer of adhesive (not shown) can be used to adhere two-compartment warming patch 90 to top surface 88 of the bag. Similarly, heat-reflective foil 98 can be applied to smooth bottom surface 102 of the bag, using a layer of adhesive (not shown). Heat-reflective foil 98 can include at least one reflective surface 100 that faces and intimately contacts bottom surface 102 of the bag.

Two-compartment warming patch 90 can include a first compartment 92, a second compartment 94, and a frangible seal 96 that keeps the contents of compartment 92 and the contents of compartment 94 separated from one another. Frangible seal 96 can comprise a thin, weak, polymeric barrier film that can be broken or ruptured by squishing the self-warming solution bag, or by otherwise manipulating warming patch 90. Warming patch 90 can be made of a material that at least partially defines a top surface, a bottom surface, ends, and sides of the patch. The material can be stronger and more durable than the material used to construct frangible seal 96. With such a construction, warming patch 90 can be manipulated, without leaking or being punctured.

Although a single two-compartment warming patch is shown in FIGS. 4 and 5, it is to be understood that two or more two-compartment warming patches can be used to affect heating of peritoneal dialysis solution within a bag. The temperature of the solution contained within peritoneal dialysis solution bag 82 can be monitored, for example, with a thermometer 104 of the same type, or similar to, thermometer 44 described in connection with FIG. 1. Once the contents of peritoneal dialysis solution bag 82 have reached an appropriate temperature, two-compartment warming patch 90 can be removed from the bag or left in contact with the bag while the temperature is monitored to make sure the solution does not get too hot. Heat-reflective foil 98 does not necessarily need to be included but can prevent heat from escaping from the solution bag and can reflect heat back into the solution.

The contents of compartment 92 and the contents of compartment 94 can be selected such that when frangible seal 96 is ruptured, the contents from the two compartments mix together to form an exothermic reaction. The sidewalls of two-compartment warming patch 90 can be durable enough to retain the contents of compartments 92 and 94, such that no restrictions are needed on what reactants can be used. The contents of each compartment can independently be liquid, gaseous, or solid. Although liquid contents are depicted in FIG. 5, it is to be understood that the separated reactants can be in any suitable form. In a first exemplary embodiment, a calcium chloride powder can be provided in compartment 92 and water can be provided in compartment 94. Upon rupturing frangible seal 96, the calcium chloride powder and water come into contact with one another and the calcium chloride reacts with water to form calcium oxide and hydrochloric acid through an exothermic reaction. The heat generated is transferred to the peritoneal dialysis solution within bag 82, thus warming the peritoneal dialysis solution.

In a second exemplary embodiment, compartment 92 can contain solid crystals of sodium acetate and compartment 94 can contain a supersaturated solution of sodium acetate. Upon rupturing a frangible seal 96, the contents of compartment 92 and the contents of compartment 94 mix together causing the rapid crystallization of solid sodium acetate, which generates heat and thus warms the peritoneal dialysis solution.

In a third exemplary embodiment, compartment 92 can contain an aqueous solution of a base, for example, a powerful base such as sodium hydroxide. Compartment 94 can contain an aqueous solution of an acid, for example, a powerful acid such as hydrogen chloride. Upon rupturing frangible seal 96, the acid and base react with one another in an exothermic reaction, forming salt and generating heat.

Although two-component reactions are described, it is to be understood that reactions between three or more components can be used to generate heat and the warming patch can comprise more than two separated compartments.

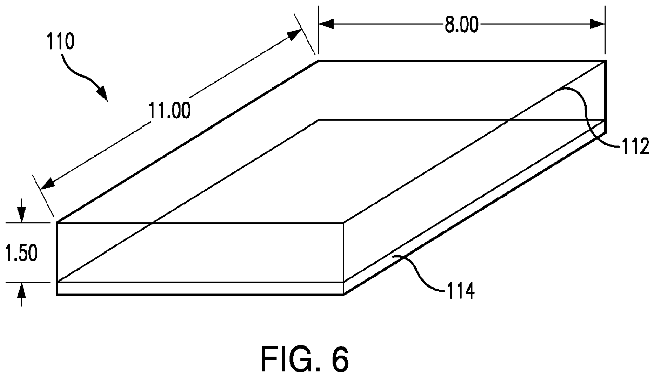

FIG. 6 is a perspective schematic view of a peritoneal dialysis solution bag 110, showing exemplary relative dimensions. A solution bag 112 is provided with a warming patch 114 in the form of a thin layer that is of the same length and width as solution bag 112 containing peritoneal dialysis solution. The equal dimensions help ensure even thermal conduction. The volume of solution that can be adequately heated by the bag can be determined by the rate and output of heat required to properly warm the solution. The bag can have a color-coded indicator showing the user the relative heat, such as blue, to show the solution is too cold, yellow, to show the solution is near range (above or below), and green, to show that the temperature of the solution is good. The temperature can be verified with a thermometer as a precaution. The color indicator can be used to indicate when it is time to take a precise measurement of temperature. Without a color indicator, multiple temperature measurements may be needed, which are blind or based on the feel of the bag. The warming patch can have a peel-away layer on its exterior to shield it from the ambient air and prevent it from prematurely reacting.

In operation, a user or patient can set the bag down with the warming patch facing upward. The patient can then peel away the protective layer on the warming patch so that a breathable membrane and thus the contents of the warming patch are exposed to air, causing a chemical reaction. Provisions such as bumps or ridges can be included in the bag design to make sure air can contact the warming patch even when its exposed surface is facing downwardly. If a two-compartment warming patch is used, the patient can instead rupture the frangible seal. The patient can then turn the bag over so that the heat generated rises into the fluid. A tray with holes in the bottom and feet, spacers, or pedestals, can be provided to elevate the peritoneal dialysis solution bag so that, even when upside down, the activatable agent can be exposed to environmental elements such as air.

The color indicator can be designed to be blue at the start. Once the indicator turns green, the temperature can be verified with a thermometer.

Once an appropriate solution temperature is verified, the patient can be instructed to deliver the solution into the peritoneal cavity within a time limit, so that the temperature of the solution will not substantially change during transfer. To prevent overheating the solution, the warming patch can be made removable, for example, as a peel-away component. The warming patch can be configured to heat the solution to any appropriate temperature, for instance, to at least 35°C (95°F), to at least 35.6°C (96°F), to at least 36.1°C (97°F), or to at least 36.7°C (98°F). In an exemplary embodiment, the warming patch can be configured to heat the solution to a temperature of, or that is a little higher than, 37°C (98.6°F). For example, constructions and amounts of warming material can be used that are configured to heat the peritoneal solution, from room temperature or 23.9°C (75°F), to about 37°C (98.6°F), or higher. Higher amounts of warming material can be used and are particularly useful in relatively cooler ambient temperature settings, such as when the peritoneal solution needs to be heated from a starting temperature of 21.1°C (70°F) or lower, for example, from a refrigerator set at 2.8°C (37°F). Many factors can influence the heating, heating rate, and final temperature of the peritoneal solution, including environmental conditions, ambient temperature, wind chill, the components in the peritoneal dialysis solution, and the like. In some cases, the warming patch can be configured to heat the solution to at least 37.8°C (100°F), at least 38.3°C (101°F), at least 38.9°C (102°F), at least 39.4°C (103°F), or higher, for example, when the bag and solution is intended for use in colder climates. Under colder conditions, such elevated temperatures and a planned overshoot of a desired temperature can result in warming the solution to within an ideal range, for example, to a temperature of from about 36.7°C (98°F) to about 37.2°C (99°F). In some cases, components of the peritoneal solution should not be heated above certain temperatures, for example, solutions containing dextrin may require avoiding temperatures of 40°C (104°F) or higher. In such circumstances, the warming patch can be configured to heat the solution to a temperature, for instance, of no higher than 39.4°C (103°F), no higher than 38.9°C (102°F), no higher than 38.3°C (101°F), or to no higher than 37.8°C (100°F). In an exemplary embodiment, the warming patch can be configured to heat the solution to a temperature of, or that is no higher than, 37°C (98.6°F). After heating, the time in which the solution should be inserted into the peritoneal cavity can be from about 1 minute to about 30 minutes, from about 5 minutes to about 20 minutes, or from about 10 minutes to about 15 minutes.

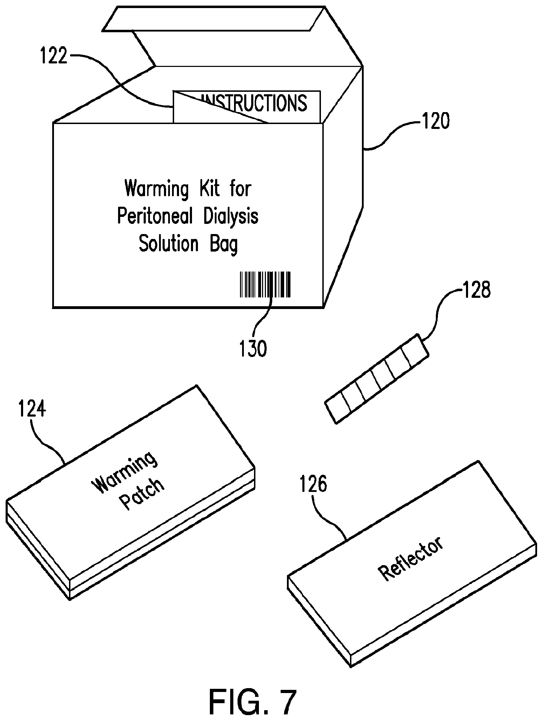

FIG. 7 is a perspective view of a kit according to one or more embodiments of the present disclosure. The kit includes a packaging box 120, an instruction sheet 122, a warming patch 124, a heat-reflective foil 126, and a color-indicator thermometer 128. Although the kit is shown with many other contents removed, it is to be understood that for storage, shipment, and sales, warming patch 124, heat-reflective foil 126, thermometer 128, and instruction sheet 122 are all placed inside box 120 and box 120 is closed. In such a packaged state, box 120 can be further sealed and/or hermetically sealed as with shrink wrap or another plastic wrapper, to prevent tampering and to minimize the chance of contamination. Each of warming patch 124, heat-reflective foil 126, and thermometer 128 can be provided with an adhesive layer for adhering the respective component to a peritoneal dialysis solution bag. The adhesive layers can each be protected by a release liner as is well known in the art of pressure sensitive adhesive labels. Packaging box 120 can be provided with a bar code 130 or another tag, marking, or indicia, that provides information indicating what type and/or volume of peritoneal dialysis solution bag can be warmed using the kit. More than one warming patch can be provided in the kit so that a greater heating capacity is available, for example, if needed in colder climates. Each warming patch 124 can be activated by exposure to environmental elements, as described in connection with the embodiments shown in FIGS. 1-3, or it can comprise a two-compartment warming patch as described in connection with FIGS. 4 and 5 herein. The kit does not necessarily have to include each of the components shown in FIG. 7 and can be as simple as a warming patch in a box, bag, envelope, or other packaging container. Whether the kit contains a thermometer is optional. Whether the kit contains a reflector is optional. Whether the instructions are provided on a separate instruction sheet is optional.

The patient can heat a peritoneal dialysis solution bag anywhere, even if they do not have a power source. The patient thus has a greater degree of independence compared to using a bag that requires heating with an electric heating pad. The patient has no need to transport a heating pad and/or cooler. The color indicator tells the patient when to check the temperature, which can prevent burning a hand as might happen if the patient touches the bag to get a general idea of how warm it is.

Depending on the volume of the dialysis solution and the chemical mix in the warming patch, the patch can heat the dialysis solution much faster than the 1.5 to 2.0 hours that it currently takes to heat a peritoneal dialysis solution bag with a heating pad. There is better thermal conduction because there is no air gap. When using a heating pad, there is an air gap between the pad and the solution bag, leading to poor heat transfer due to convection and conduction, as opposed to heat transfer by conduction only when intimate contact is made.

When an amount, concentration, or other value or parameter is given as either a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

Other embodiments of the present invention will be apparent to those skilled in the art from consideration of the present specification and practice of the present invention disclosed herein. It is intended that the present specification and examples be considered as exemplary only with a true scope of the invention being indicated by the following claims.

## Claims

1. A self-warming peritoneal dialysis solution bag (20) containing a peritoneal dialysis solution, the bag having an outer surface (38), a non-electric, warming patch (32, 34) affixed to the outer surface, and a drainage port (46), wherein the non-electric, warming patch (32, 34) comprises a breathable membrane (54), the warming patch containing an activatable agent (58) that undergoes an exothermic reaction when activated with air, and the warming patch retains a sufficient amount of activatable agent to generate enough heat, upon activation, to warm the contents of the bag from 23.9°C (75°F) to a temperature of from 35°C (95°F) to not higher than 39.4°C (103°F) such that the peritoneal dialysis solution is within an acceptable temperature range for transfer into a peritoneal cavity, wherein at least one of the non-electric, warming patch (32, 34) comprises a removable, resealable protective cover (36) that prevents activation of the activatable agent until the removable, resealable protective cover (36) is removed from contact with the breathable membrane and terminates activation when reapplied to the breathable membrane, and wherein the warming peritoneal dialysis solution bag (20) further contains a temperature indicator (44) affixed to the bag, the temperature indicator comprising a color indicator, the color indicator comprising a first color indicator configured to indicate when the solution in the bag is at a suitable temperature, and one or more other color indicators configured to indicate when the solution in the bag is not hot enough or is too hot.

2. The self-warming peritoneal dialysis solution bag of claim 1, wherein the removable, resealable protective cover (36) is adhered to the non-electric, warming patch (32, 34) by a releasable pressure sensitive adhesive (40).

3. The self-warming peritoneal dialysis solution bag of claim 1, wherein the non-electric, warming patch (32, 34) is adhered to the outer surface (38) of the bag.

4. The self-warming peritoneal dialysis solution bag of claim 1, wherein the activatable agent comprises an oxidizable metal.

5. The self-warming peritoneal dialysis solution bag of claim 1, wherein the non-electric, warming patch (32, 34) has one patch ((32) or (34)) that comprises two compartments (92, 94) separated by a frangible seal (96) and wherein preferably a first compartment (92) of the two compartments comprises a first reactant, a second compartment (94) of the two compartments comprises a second reactant, and the first and second reactants, when in contact with each other, exothermically react; or wherein the non-electric, warming patch (32, 34) comprises two compartments separated by a frangible seal (96), and at least one of the compartments contains a sodium acetate reagent.

6. The self-warming peritoneal dialysis solution bag of claim 1, wherein a heat-reflective material (98) is in contact with an outer surface of the bag, wherein the heat-reflective material (98) preferably comprises a heat-reflective foil adhered to the outer surface of the bag or a metal foil, and wherein the heat-reflective foil is preferably in contact with a first side of the bag, the heat-reflective foil comprises a reflective surface that faces the inside of the bag, and the non-electric, warming patch is disposed on a second side of the bag, opposite the first side.

7. A thermally-insulated, self-warming, peritoneal dialysis solution bag comprising the self-warming peritoneal dialysis solution bag (20) of claim 1, and a bag holder configured to be worn by a user, the bag holder comprising a pouch configured to accommodate the self-warming peritoneal dialysis solution bag (20), wherein the pouch comprises heat-insulating material configured to thermally insulate the peritoneal dialysis solution bag, and wherein the bag holder preferably comprises a vest with through-holes for a user's arms, or a belt.

8. The self-warming peritoneal dialysis solution bag of claim 1, wherein the activatable agent comprises an oxidizable metal, said oxidizable metal comprising iron, an iron oxide material, or a combination thereof.

9. The self-warming peritoneal dialysis solution bag of claim 1, wherein the non-electric, warming patch comprises a removable, peel-away component configured to be peeled-away to prevent overheating of the peritoneal dialysis solution.

## Patentansprüche

1. Selbstwärmender Peritonealdialyse-Lösungsbeutel (20), der eine Peritonealdialyselösung enthält, wobei der Beutel eine Außenfläche (38), ein nicht-elektrisches, wärmendes Pflaster (32, 34), das an der Außenfläche befestigt ist, und eine Drainageöffnung (46) aufweist, wobei das nicht-elektrische, wärmende Pflaster (32, 34) eine atmungsaktive Membran (54) umfasst, wobei das wärmende Pflaster ein aktivierbares Mittel (58) enthält, das eine exotherme Reaktion eingeht, wenn es mit Luft aktiviert wird, und das wärmende Pflaster eine ausreichende Menge des aktivierbaren Mittels zurückhält, um bei Aktivierung genügend Wärme zu erzeugen, um den Inhalt des Beutels von 23,9°C (75°F) auf eine Temperatur von 35°C (95°F) bis nicht höher als 39,4°C (103°F) zu erwärmen, so dass die Peritonealdialyselösung innerhalb eines akzeptablen Temperaturbereichs zur Übertragung in eine Peritonealhöhle liegt, wobei mindestens eines der nicht-elektrischen, wärmenden Pflaster (32, 34) eine entfernbare, wiederverschließbare Schutzabdeckung (36) umfasst, die die Aktivierung des aktivierbaren Mittels verhindert, bis die entfernbare, wiederverschließbare Schutzabdeckung (36) vom Kontakt mit der atmungsaktiven Membran entfernt wird und die Aktivierung beendet, wenn sie wieder auf die atmungsaktive Membran aufgebracht wird, und
wobei der wärmende Peritonealdialyse-Lösungsbeutel (20) ferner einen an dem Beutel befestigten Temperaturindikator (44) enthält, wobei der Temperaturindikator einen Farbindikator umfasst, wobei der Farbindikator einen ersten Farbindikator umfasst, der so konfiguriert ist, dass er anzeigt, wenn die Lösung in dem Beutel eine geeignete Temperatur hat, und einen oder mehrere andere Farbindikatoren, die so konfiguriert sind, dass sie anzeigen, wenn die Lösung in dem Beutel nicht heiß genug oder zu heiß ist.

2. Selbstwärmender Peritonealdialyse-Lösungsbeutel nach Anspruch 1, wobei die abnehmbare, wiederverschließbare Schutzabdeckung (36) an dem nicht-elektrischen, wärmenden Pflaster (32, 34) durch einen ablösbaren Haftkleber (40) angeklebt ist.

3. Selbstwärmender Peritonealdialyse-Lösungsbeutel nach Anspruch 1, wobei das nicht-elektrische, wärmende Pflaster (32, 34) an der Außenfläche (38) des Beutels haftet.

4. Selbstwärmender Peritonealdialyse-Lösungsbeutel nach Anspruch 1, wobei das aktivierbare Mittel ein oxidierbares Metall umfasst.

5. Selbstwärmender Peritonealdialyse-Lösungbeutel nach Anspruch 1, wobei das nicht-elektrische, wärmende Pflaster (32, 34) ein Pflaster ((32) oder (34)) aufweist, das zwei Kammern (92, 94) umfasst, die durch eine zerbrechliche Dichtung (96) getrennt sind, und wobei vorzugsweise eine erste Kammer (92) der beiden Kammern einen ersten Reaktanten umfasst, eine zweite Kammer (94) der beiden Kammern einen zweiten Reaktanten umfasst, und der erste und der zweite Reaktant, wenn sie miteinander in Kontakt sind, exotherm reagieren; oder wobei das nicht-elektrische, wärmende Pflaster (32, 34) zwei Kammern umfasst, die durch eine zerbrechliche Dichtung (96) getrennt sind, und mindestens eine der Kammern ein Natriumacetat-Reagens enthält.

6. Selbstwärmender Peritonealdialyse-Lösungsbeutel nach Anspruch 1, wobei ein wärmereflektierendes Material (98) mit einer Außenfläche des Beutels in Kontakt ist, wobei das wärme-reflektierende Material (98) vorzugsweise eine wärme-reflektierende Folie, die an der Außenfläche des Beutels haftet, oder eine Metallfolie umfasst, und wobei die wärmereflektierende Folie vorzugsweise mit einer ersten Seite des Beutels in Kontakt ist, die wärmereflektierende Folie eine reflektierende Oberfläche umfasst, die der Innenseite des Beutels zugewandt ist, und das nicht-elektrische, wärmende Pflaster auf einer zweiten Seite des Beutels, gegenüber der ersten Seite, angeordnet ist.

7. Wärmeisolierter, selbstwärmender Peritonealdialyse-Lösungsbeutel, umfassend den selbstwärmenden Peritonealdialyse-Lösungsbeutel (20) nach Anspruch 1 und einen Beutelhalter, der so konfiguriert ist, dass er von einem Benutzer getragen wird, wobei der Beutelhalter einen Beutel umfasst, der so konfiguriert ist, dass er den selbstwärmenden Peritonealdialyse-Lösungsbeutel (20) aufnimmt, wobei der Beutel wärmeisolierendes Material umfasst, das so konfiguriert ist, dass es den Peritonealdialyse-Lösungsbeutel thermisch isoliert, und wobei der Beutelhalter vorzugsweise eine Weste mit Durchgangslöchern für die Arme eines Benutzers oder einen Gürtel umfasst.

8. Selbstwärmender Peritonealdialyse-Lösungsbeutel nach Anspruch 1, wobei das aktivierbare Mittel ein oxidierbares Metall umfasst, wobei das oxidierbare Metall Eisen, ein Eisenoxidmaterial oder eine Kombination davon umfasst.

9. Selbstwärmender Peritonealdialyse-Lösungsbeutel nach Anspruch 1, wobei das nicht-elektrische, wärmende Pflaster eine entfernbare, abziehbare Komponente umfasst, die so konfiguriert ist, dass sie abgezogen werden kann, um eine Überhitzung der Peritonealdialyselösung zu verhindern.

## Revendications

1. Sac auto-chauffant de solution de dialyse péritonéale (20) contenant une solution de dialyse péritonéale, le sac ayant une surface externe (38), un patch chauffant non électrique (32, 34) apposé à la surface externe, et un orifice de drainage (46), le patch chauffant non électrique (32, 34) comprenant une membrane respirante (54), le patch chauffant contenant un agent pouvant être activé (58) qui subit une réaction exothermique lorsqu'activé avec de l'air, et le patch chauffant conservant une quantité suffisante d'agent pouvant être activé pour générer suffisamment de chaleur, lors de l'activation, pour chauffer les contenus du sac de 23,9°C (75°F) à une température de 35°C (95°F) à pas plus de 39,4°C (103°F) de sorte que la solution de dialyse péritonéale se situe à l'intérieur d'une plage de températures acceptable pour le transfert dans une cavité péritonéale, où au moins l'un du patch chauffant non électrique (32, 34) comprend un couvercle protecteur refermable, amovible (36) qui empêche l'activation de l'agent pouvant être activé jusqu'à ce que le couvercle protecteur refermable, amovible (36) soit retiré du contact avec la membrane respirante et achève l'activation lorsque réappliqué à la membrane respirante, et
où le sac chauffant de solution de dialyse péritonéale (20) contient en outre un indicateur de température (44) apposé au sac, l'indicateur de température comprenant un indicateur coloré, l'indicateur coloré comprenant un premier indicateur coloré configuré pour indiquer lorsque la solution dans le sac se trouve à une température appropriée, et un ou plusieurs autres indicateurs colorés configurés pour indiquer lorsque la solution dans le sac n'est pas suffisamment chaude ou est trop chaude.

2. Sac auto-chauffant de solution de dialyse péritonéale selon la revendication 1, dans lequel le couvercle protecteur refermable, amovible (36) adhère au patch chauffant non électrique (32, 34) par un adhésif sensible à la pression pouvant être décollé (40).

3. Sac auto-chauffant de solution de dialyse péritonéale selon la revendication 1, dans lequel le patch chauffant non électrique (32, 34) adhère à la surface externe (38) du sac.

4. Sac auto-chauffant de solution de dialyse péritonéale selon la revendication 1, dans lequel l'agent pouvant être activé comprend un métal oxydable.

5. Sac auto-chauffant de solution de dialyse péritonéale selon la revendication 1, dans lequel le patch chauffant non électrique (32, 34) présente un patch ((32) ou (34)) qui comprend deux compartiments (92, 94) séparés par un joint sécable (96) et où préférablement un premier compartiment (92) des deux compartiments comprend un premier réactif, un second compartiment (94) des deux compartiments comprend un second réactif, et le premier et le second réactif, lorsqu'ils se trouvent en contact l'un avec l'autre, réagissent au plan exothermique ; ou où le patch chauffant non électrique (32, 34) comprend deux compartiments séparés par un joint sécable (96), et au moins l'un des compartiments contient un réactif acétate de sodium.

6. Sac auto-chauffant de solution de dialyse péritonéale selon la revendication 1, dans lequel un matériau thermo-réfléchissant (98) se trouve en contact avec une surface externe du sac, où le matériau thermo-réfléchissant (98) comprend préférablement un feuil thermo-réfléchissant qui adhère à la surface externe du sac ou d'un feuil métallique, et où le feuil thermo-réfléchissant se trouve préférablement en contact avec un premier côté du sac, le feuil thermo-réfléchissant comprend une surface réfléchissante qui fait face vers l'intérieur du sac, et le patch chauffant non électrique est disposé sur un second côté du sac, opposé au premier côté.

7. Sac thermiquement isolé, auto-chauffant, de solution de dialyse péritonéale comprenant le sac auto-chauffant de solution de dialyse péritonéale (20) selon la revendication 1, et un support de sac configuré pour être porté par un-e utilisateur-trice, le support de sac comprenant une poche configurée pour loger le sac auto-chauffant de solution de dialyse péritonéale (20), où la poche comprend le matériau thermo-isolant configuré pour isoler thermiquement le sac de solution de dialyse péritonéale, et où le support de sac comprend préférablement une veste avec des trous traversants pour les bras d'un-e utilisateur-trice, ou une ceinture.

8. Sac auto-chauffant de solution de dialyse péritonéale selon la revendication 1, dans lequel l'agent pouvant être activé comprend un métal oxydable, ledit métal oxydable comprenant du fer, un matériau oxyde de fer, ou une combinaison de ceux-ci.

9. Sac auto-chauffant de solution de dialyse péritonéale selon la revendication 1, dans lequel le patch chauffant non électrique comprend un composant amovible à décoller configuré pour être décollé pour empêcher le surchauffage de la solution de dialyse péritonéale.
